Europäisches Patentamt

**European Patent Office**

Office européen des brevets

(19)

(11) Publication number: **0 024 855**
**B1**

(12) EUROPEAN PATENT SPECIFICATION

(45) Date of publication of patent specification: **27.06.84**

(51) Int. Cl.³: **A 61 H 33/00, A 47 K 3/10**

(21) Application number: **80302793.7**

(22) Date of filing: **13.08.80**

(54) Sensory isolation tanks.

(30) Priority: **29.08.79 GB 7929849**
**01.02.80 GB 8003539**

(43) Date of publication of application:
**11.03.81 Bulletin 81/10**

(45) Publication of the grant of the patent:
**27.06.84 Bulletin 84/26**

(84) Designated Contracting States:
**AT CH DE FR LI NL SE**

(56) References cited:
**BE - A - 873 174**
**US - A - 1 636 401**
**US - A - 3 946 200**

(73) Proprietor: **Allcock, John Anthony**
**36 Saville Crescent Ashford Common**
**Ashford Middlesex (GB)**

(72) Inventor: **Allcock, John Anthony**
**36 Saville Crescent Ashford Common**
**Ashford Middlesex (GB)**

(74) Representative: **Saunders, Henry Edmund et al,**
**Baron & Warren 18 South End Kensington**
**London W8 5BU (GB)**

Courier Press, Leamington Spa, England.

## Description

The present invention relates to sensory isolation tanks, that is tanks in which a person can remain isolated, floating in a supporting liquid and substantially free of all external sensory stimuli or being stimulated only in a selected sense or senses. Such tanks have a very beneficial effect on the human psyche and are a very real incentive to concentration and conducive to mental well-being and physical relaxation. A difficulty met in such tanks is that the person using the tank has a tendency to drift towards a tank wall and consequently touch the wall and thereby giving him or her the unwanted sensory stimulus of touch which can reduce the benefits so far acquired during the sensor isolation.

Previously attempts have been made to meet this difficulty by making tanks relatively large in relation to the human body so as to try and minimise unwanted contact with the tank walls. However this increase in size brings with it attendant disadvantages such as increased cost and difficulty in finding suitable tank locations.

The present invention has for an object to alleviate these disadvantages.

Accordingly the present invention consists in a sensory isolation tank having one or more heating elements for heating a flotation solution contained in the tank during its use, a temperature sensor for sensing the temperature of the flotation solution, and temperature control means for maintaining the temperature of the flotation solution at a present level by controlling the supply of energy to said heating element or elements, and wherein said heating element or elements are adapted to heat the peripheral edges of the flotation solution thereby to cause convection currents in the flotation solution which tend to maintain the user of the tank in the centre thereof.

The provision of such a centering action for tank users enables the tank dimensions to be reduced.

In order that the present invention may be more readily understood an embodiment thereof will now be described by way of example with reference to the accompanying drawings, in which:—

Figure 1 is a section through a sensor deprivation tank according to the present invention,

Figure 2 is a diagrammatic section through the tank of Figure 1,

Figures 3a and b are circuit diagrams of a temperature control circuit for use with the tank of Figures 1 and 2, and

Figure 4 is a circuit diagram of the integrated circuit switch used in the temperature control circuit of Figures 3a and 3b.

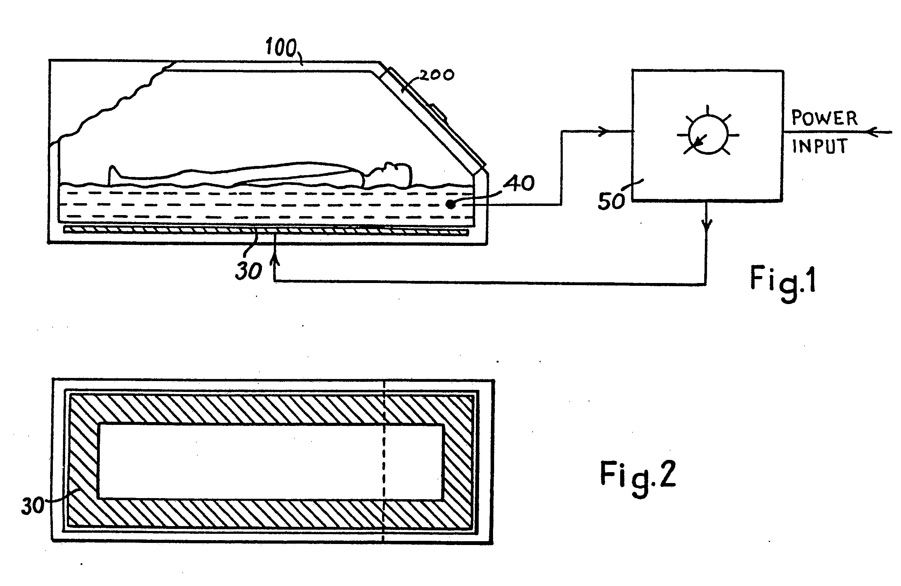

Referring now to the drawings, Figures 1 and 2 show a sensory isolation tank 100 which is substantially oblong in shape but which has an inclined entry door 200.

The tank is made from fibreglass reinforced plastics panels which slot together to form a rigid unit approximately 84" (213 cm) long, 37" (94 cm) wide and 40" (102 cm) high. Each panel may be flanged so that the panels can be bolted together. It is conceivable that the main body of the tank could be a single moulding. The tank includes an electrical heating element 30 for maintaining the temperature of the liquid in the tank at a preset level and the liquid temperature is sensed by a sensor 40 which in the present embodiment is a thermistor. The heating element 30 may be a relatively heavy duty cable spirally wound and approximately 80 ft (24.4 m) in length. The cable may be received in a recess in the base of the tank and encapsulated in synthetic resin. Naturally other known types of heating elements may be used. The heating element or elements are controlled by a control arrangement indicated at 50 and which will be described in greater detail hereinafter.

In use the tank contains sufficient water for a user of the tank to float and this water contains additional sea-salt or epsom salts in such a quantity that the specific gravity of the water is considerably raised enabling a user to float effortlessly in a supine position. A typical depth of water may be from 8" (20 cm) to 9" (23 cm).

The temperature control arrangement in use maintains the temperature of the water very precisely. Normally the temperature will be maintained at about 94.2°F/34.6°C although small variations above and below this value is permissible. However, the tank should not be used if the temperature is above 95°F/35°C or below 91°F/32.8°C.

It is important that during use of the tank, the user remain floating in the centre of the tank and does not touch the walls of the tank. This is because, as mentioned before, the act of touching the walls causes unwanted sensory stimulation. Thus in the present embodiment a proportional heating system is used by means of which the heating element 30 supplies to the tank just sufficient energy to maintain the temperature of the tank at the desired level by compensating for the heat losses from the tank which occur. Thus there is always a certain quantity of power being applied to the heating element 30 to maintain the preselected temperature and to correct to heat losses from the tank. This condition creates convection currents in the flotation solution in the tank. As the heating element 3 is placed around the perimeter of the tank, the solution adjacent to the element 30, rises to the surface to be replaced by slightly cooler solution from the centre of the tank thereby creating a cycling flow of solution currents in which the upper surface layers flow towards the tank centre. As the user of the tank is floating on or in the upper surface layers of the high density solution, his or her physical position with respect to the tank perimeter always tends to remain in the centre of the tank under the influence of these convection currents.

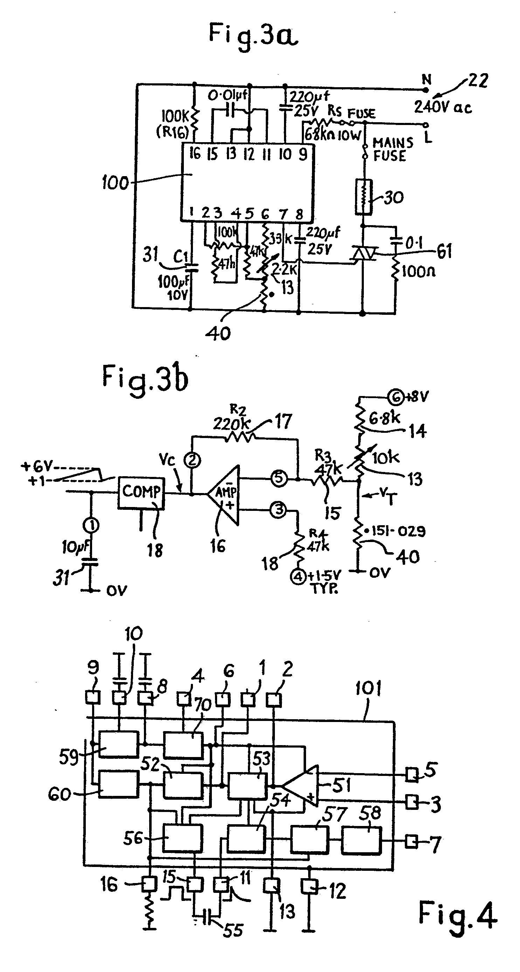

Figures 3a and 3b are circuit diagrams of the temperature control system with Figure 3a showing the overall arrangement. Thus in Figure 3 an integrated circuit zero voltage switch 101 is connected to the gate electrode of a triac 61 which is arranged to gate the supply of electrical energy to the tank heating element 30. The zero voltage switch 101 may comprise an integrated circuit module of the kind marketed by R. S. Components Limited under the number 305-800. Power is supplied by standard A.C. mains as indicated at 22 and the temperature sensor formed by a thermistor 40 is also shown in this circuit. When the thermistor 40 detects that the temperature in the solution in the tank is low the gate of triac 61 is opened by a signal from switch 101 to supply current to the heating element 30. The triac 61 is only opened for a period sufficient to maintain the flotation solution in the tank at the required temperature by compensating for the heat energy lost.

Figure 4 shows the switch 101 in greater detail and from this figure it can be seen that switch 100 comprises an amplifier 51 having a non-inverting input from terminal pin 3 of the switch, and an inverting input from terminal pin 5. The output of amplifier 51 is connected to terminal pin 2, whilst terminal pin 1 receives a ramp voltage from a ramp generator 52. Switch 101 also includes a comparator 53 connected between the output of amplifier 52 and ramp generator 52, and a chopper circuit 54 connected to terminal pin 11, terminal pin 11 being coupled to terminal pin 15 via a capacitor 55 so as to transmit to the chopper circuit 54 a signal corresponding to the leading edge of an output pulse from a control logic unit 56 connected to terminal 15. The output of chopper circuit 54 is connected to the input of an output logic stage 57 which receives current from terminal pin 16 of the switch. The output of output logic stage 57 is connected to a gate pulse stage 58 which provides output pulses on terminal pin 7 which are supplied to the gate of triac 61 to control its conduction periods. The A.C. supply is connected to terminal pin 9, is clipped and rectified in a clipping and rectifying circuit 59 and supplied to a voltage regulator 70 which supplies a stabilised D.C. reference voltage to terminal pin 6, and also a D.C. voltage to terminal pin 4. The terminal pins 8 and 10 are respectively for positive rectified supply and negative rectified supply. The t;erminal pin 9 is also supplied to a zero crossing detector circuit 60 the output of which is supplied both to the ramp generator 52, the control logic circuit 55 and the output logic circuit 56. Referring again to Figure 3a it can be seen that the terminal pins 8 and 10 are connected to the N line of the A.C. mains by electrolytic capacitors 62, 62'. Terminal pins 12 and 13 are ground terminals.

The desired temperature can be set, within limits by a variable resistor 33. Figure 3b shows in greater detail how the conduction periods of triac 61 are controlled. Thus, in Figure 3b the same numerals are used to indicate the various terminal pins of the zero voltage switch 101. From Figure 3b it can be seen that thermistor 40 is located in one arm of a voltage divider connected between earth and a potential of +8 volts at terminal pin 6, The other arm of the voltage divider consists of variable resistor 63 and a fixed resistor 64.

The central tapping of the voltage divider is taken via resistor 65 or $R_3$ ohms to terminal pin 5 which is the inverting input of amplifier 51 and also to the terminal pin 2 via a resistor 17 of $R_2$ ohms. The non-inverting input of amplifier 51 is provided with a reference voltage derived from a +1.5 volts potential at terminal pin 4, pin 4 being connected to the pin 3 by a resistance 18 which is of the same value as resistance 65.

Thus a voltage $V_c$ appears at pin 2 which is approximately equal to

$$\frac{R2}{R3}(1.5-V_T),$$

where $V_T$ is dependent on the resistance of thermistor 4.

This voltage is compared with a ramp voltage by comparator 52 so as to give output signals at terminal pin 7. These output signals are connected to the gate of triac 61 to control firing of the latter in a manner to be discussed. The ramp voltage is generated by the charging and discharging of a capacitor 31 connected to terminal pin 2 of the switch 101.

When terminal pin 2 of the switch 100 is at a lower voltage than the bottom of the ramp voltage, the heating element 30 is heated permanently so that the flotation solution is heated relatively rapidly to the desired temperature. When the pin 2 is at a voltage higher than the maximum voltage of the ramp the triac 61 is off so that the heating element 30 receives no current. Voltages on pin 2 lying between the bottom and top of the ramp produce bursts of power to the heating element 30 proportional to the value of the voltage on pin 2 with regard to the ramp. Thus triac 61 will be opened for a period dependent on the voltage of pin 2. For correct operation of this temperature control arrangement the ramp period should be much shorter than the thermal constant of the tank but long enough to accommodate many cycles of the mains voltage.

## Claims

1. A sensory isolation tank (100) having one or more heating elements (30) for heating a flotation solution contained in the tank during its use, a temperature sensor (40) for sensing the temperature of the flotation solution, and temperature control means (50) for maintaining the temperature of the flotation solution at a preset level by controlling the supply of energy to said heating element or elements, and characterised in that said heating element or elements (30) are adapted to heat the peripheral edges of the flotation solution thereby to cause convection currents in the flotation solution which tend to maintain the user of the tank in the centre thereof.

2. A sensory isolation tank as claimed in claim 1, wherein said temperature control means include means (52) for generating a ramp voltage, means (51) for comparing said ramp voltage with a signal proportional to the temperature sensed by said temperature sensing means, and means (57) responsive to said comparison for controlling the supply of energy to said heating element or elements.

3. A sensory isolation tank as claimed in claim 2, wherein the means for controlling the supply of energy comprise a semiconductor switching device (11) having a gate electrode to which the output of said temperature control means is connected.

4. A sensory isolation tank as claimed in claim 3, wherein said temperature control means comprise an integrated circuit switch (10) connected to a thermistor which forms the temperature sensing means, and connectible to a source of A.C. current, the energy supply control means comprising a triac.

## Patentansprüche

1. Den Sinnesapparat isolierender Behälter (100) mit einem oder mehreren Heizelementen (30) zum Erwärmen einer in dem Behälter enthaltenen Aufschwemmlösung während der Benutzung, einem Temperaturfuler (40), der die Temperatur der Aufschwemmlösung wahrnimmt, und einer Temperaturreglereinrichtung (52), die durch Steuern der Energiezufuhr zu dem oder den Heizelementen die Temperatur der Aufschlemmlösung auf einem vorgegebenen Wert hält, dadurch gekennzeich- ·net, daß das Heizelement oder die Heizelemente (30) geeignet ist/sind die Umfangsränder der Aufschwemmlösung zu erwärmen, um dadurch Konvektionsströme in der Aufschwemmlösung zu verursachen, die bestrebt sind, den Benutzer des Behälters in der Behältermitte zu halten.

2. Den Sinnesapparat isolierender Behälter nach Anspruch 1, bei dem die Temperaturreglereinrichtung eine Einrichtung (52) zum Erzeugen einer Sägezahnspannung, eine Einrichtung (51) zum Vergleichen der Sägezahnspannung mit einem zu der von der Temperaturfühlereinrichtung wahrgenommenen Temperatur proportionalen Signal sowie eine Einrichtung (57) aufweist, die in Abhängigkeit von dem Vergleich die Energiezufuhr zu dem Heizelement oder den Heizelementen steuert.

3. Den Sinnesapparat isolierender Behälter nach Anspruch 2, bei dem die Einrichtung zum Steuern der Energiezufuhr ein Halbleiter-Schaltelement (11) mit einer Torelektrode aufweist, mit der der Ausgang der Temperaturreglereinrichtung verbunden ist.

4. Den Sinnesapparat isolierender Behälter nach Anspruch 3, bei dem die Temperaturreglereinrichtung einen IC-Schalter (10) aufweist, der mit einem die Temperaturfühlereinrichtung bildenden Thermistor verbunden und an eine Wechselstromquelle anschließbar ist, wobei die Einrichtung zum Steuern der Energiezufuhr einen Triac aufweist.

## Revendications

1. Enceinte d'isolement sensoriel (100) ayant un ou plusieurs éléments chauffants (30) pour chauffer une solution de flottation contenue dans l'enceinte pendant son utilisation, un capteur de température (40) pour capter la température de la solution de flottation, et un organe de commande de la température (50) pour maintenir la température de la solution de flottation à un niveau prédéterminé en commandant la fourniture d'énergie audit élément (ou éléments) chauffant, caractérisée en ce que l'élément (ou les éléments) chauffant (30) est adapté à chauffer les bords périphériques de la solution de flottation de façon à créer des courants de convection dans la solution de flottation qui tendent à maintenir l'utilisateur de l'enceinte au centre de celle-ci.

2. Enceinte d'isolement sensoriel selon la revendication 1, caractérisée en ce que l'organe de commande de température (50) comprend des moyens (52) pour engendrer une tension en rampe, des moyens pour comparer ladite tension en rampe à un signal proportionnel à la température captée par le capteur de température, et des moyens (57) sensibles à ladite comparaison pour commander la fourniture d'énergie audit ou auxdits éléments de chauffage.

3. Enceinte d'isolement sensoriel selon la revendication 2, caractérisée en ce que les moyens pour commander la fourniture d'énergie comprennent un dispositif commutateur (11) à semiconducteur ayant une électrode porte à laquelle est connectée la sortie dudit organe de commande de température.

4. Enceinte d'isolement sensoriel selon la revendication 3, caractérisée en ce que l'organe de commande de température comporte un commutateur (10) à circuit intégré connecté à un thermistor qui forme l'organe capteur de température, et connectable à une source de courant alternatif, les moyens pour commander la fourniture d'énergie comprenant une triac.

100

200

POWER INPUT

40

50

Fig.1

30

0024855

30

Fig.2

# Fig.3a

# Fig.3b

# Fig.4